# FASCICULE DE BREVET EUROPEEN

(11) **EP 1 125 940 B1**
(45) Date de publication et mention de la délivrance du brevet: **15.01.2003**
(21) Numéro de dépôt: 01400407.1
(22) Date de dépôt: 16.02.2001
(51) Int. Cl.: C07F 9/60, A61K 31/675, A61P 25/00

(54) **Dérivés d'acides aryle ou hétéroaryle quinolinylphosphoniques, leur procédé de préparation et les compositions pharmaceutiques qui les contiennent**
Derivate von Aryl- oder Heteroaryl-Quinolinylphosphonsäuren, Verfahren zu ihrer Herstellung und sie enthaltende Arzneimittel
Derivatives of aryl or heteroaryl quinolinylphosphonic acids, process for their preparation and pharmaceutical compositions containing them

(30) Priorité: 18.02.2000 FR 0002013
(43) Date de publication de la demande: 22.08.2001
(73) Titulaire: LES LABORATOIRES SERVIER, 92200 Neuilly sur Seine (FR)
(72) Inventeur: Cordi, Alex, 92150 Suresnes (FR); Desos, Patrice, 92400 Courbevoie (FR); Lestage, Pierre, 78170 La Celle Saint Cloud (FR)

(56) Documents cités:
- EP-A- 0 542 609
- EP-A- 0 640 612
- EP-A- 0 818 449
- US-A- 5 342 946

## Description

La présente invention concerne de nouveaux dérivés d'acides aryle ou hétéroaryle quinolinyl phosphoniques, leur procédé de préparation et les compositions qui les contiennent.

L'art antérieur décrit des composés capables de s'opposer aux effets excitateurs et toxiques des aminoacides excitateurs (AAE) en bloquant l'activation initiale du récepteur à l'AMPA (acide α-amino-3-hydroxy-5-méthyl-4-isoxazole-propionique)/kainate (EP0640612). Leur utilité est ainsi reconnue pour inhiber les phénomènes pathologiques, notamment neurotoxiques liés à l'hyperactivation des voies de neurotransmission aux aminoacides excitateurs.

La demanderesse a découvert de nouveaux composés à structure originale qui présentent des propriétés d'antagonistes non-NMDA plus puissants que ceux de l'art antérieur. Ces composés sont donc nouveaux et sont de puissants agents thérapeutiques potentiels pour le traitement aigu mais également chronique des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

La présente invention concerne plus particulièrement les composés de formule (I) : 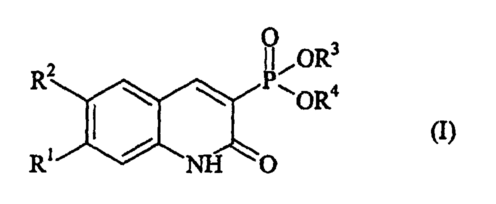 dans laquelle :
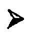 R¹ représente un atome d'halogène ou un groupement trifluorométhyle,
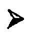 R² représente un groupement aryle ou hétéroaryle,
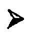 R³ et R⁴, identiques ou différents, représentent un atome d'hydrogène ou un groupement alkyle, cycloalkyle, aryle, arylalkyle ou un groupement 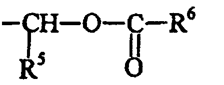 (dans lequel R⁵ et R⁶, identiques ou différents, représentent un atome d'hydrogène ou un groupement alkyle, cycloalkyle ou aryle),
étant entendu que :
- par alkyle on entend un groupement alkyle contenant 1 à 6 atomes de carbone, linéaire ou ramifié,
- par cycloalkyle on entend un groupement alkyle cyclique contenant 3 à 8 atomes de carbone,
- par aryle on entend les groupements phényle, naphtyle ou biphényle, ces groupements pouvant être non substitués ou substitués par 1 à 3 groupements choisis parmi alkyle, cycloalkyle, alkoxy, polyhalogénoalkyle, cyano, nitro, amino, alkylamino, dialkylamino, SO₂NR⁷R⁸ (où R⁷ et R⁸, identiques ou différents, représentent un atome d'hydrogène ou un groupement alkyle, cycloalkyle ou aryle) ou atomes d'halogène,
- par hétéroaryle on entend tout groupement aromatique mono ou bicyclique contenant 5 à 10 sommets et contenant 1 à 3 hétéroatomes choisis parmi oxygène, azote et soufre, l'hétéroaryle pouvant être rattaché au noyau benzénique qui le porte par un atome de carbone ou par un atome d'azote lorsqu'il en possède un et pouvant être non substitué ou substitué par 1 à 3 groupements choisis parmi alkyle, cycloalkyle, alkoxy, polyhalogénoalkyle, cyano, nitro, amino, alkylamino, dialkylamino, SO₂NR⁷R⁸ (où R⁷ et R⁸ sont tels que définis précédemment), ou atomes d'halogène,
leurs énantiomères et diastéréoisomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels R¹ représente un atome de chlore ou un groupement CF₃.

Les groupements R² préférés sont le phényle, le naphtyle ou le biphényle, ces groupements étant non substitués ou substitués et plus particulièrement par un atome d'halogène ou un groupement CF₃, NO₂, alkyle ou SO₂NH₂,
ou les groupements pyrrole ou thiophène.

Plus préférentiellement, l'invention concerne les composés de formule (I) pour lesquels R³ et R⁴ représentent simultanément un atome d'hydrogène.

Encore plus particulièrement, l'invention concerne les composés de formule (I) qui sont :
* l'acide {7-chloro-2-oxo-6-[4-(trifluorométhyl}phényl]-1,2-dihydro-3-quinolinyl} phosphonique,
* l'acide [7-chloro-6-(4-méthylphényl)-2-oxo-1,2-dihydro-3-quinolinyl]phosphonique.

L'invention s'étend également au procédé de préparation des composés de formule (I) caractérisé en ce que l'on utilise comme produit de départ le composé de formule (II) : 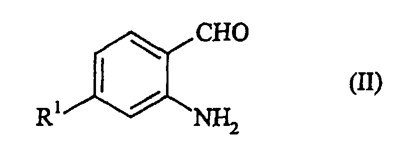 dans laquelle R¹ est tel que défini dans la formule (I), sur lequel on condense en présence de base comme la pyridine par exemple un composé de formule (III) : 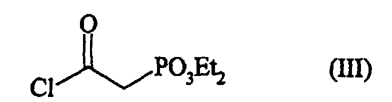 pour conduire au composé de formule (IV) : 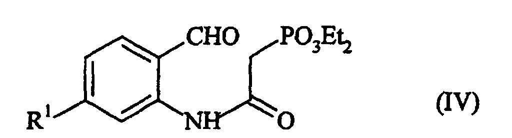 dans laquelle R¹ est tel que défini précédemment,
que l'on cyclise en présence d'une quantité catalytique de pipéridine pour obtenir le composé de formule (V) 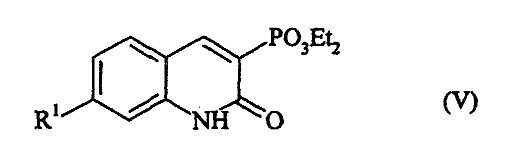 dans laquelle R¹ est défini comme précédemment,
que l'on soumet à un mélange d'acide nitrique et d'acide sulfurique pour conduire au composé de formule (VI) : 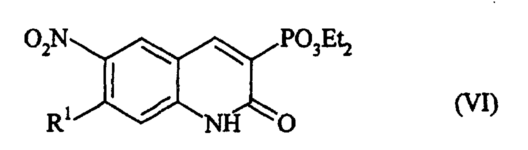 dans laquelle R¹ est tel que défini précédemment,
qui est réduit en utilisant du charbon palladié en présence d'hydrogène ou du Fer en milieu hydroalcoolique pour conduire au composé de formule (VII) 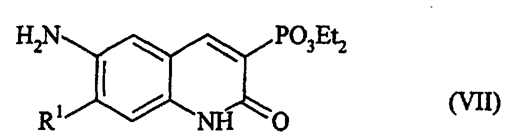 dans laquelle R¹ est défini comme précédemment,
- que l'on transforme par action de NaNO₂ et HBF₄ en sel fluoroborate de diazonium correspondant de formule (VIII) : 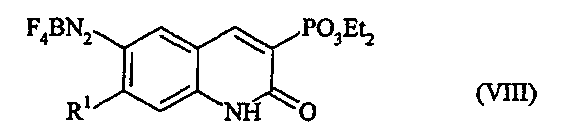
dans laquelle R¹ est tel que défini précédemment,
sur lequel on condense en présence de palladium un dérivé de l'acide boronique de formule (IX) : 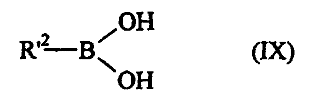 dans laquelle R'² représente un groupement aryle ou hétéroaryle tels que définis dans la formule (I), le groupement hétéroaryle étant rattaché à l'atome de bore par un atome de carbone,
pour conduire au composé de formule (I/a), cas particulier des composés de formule (I) : 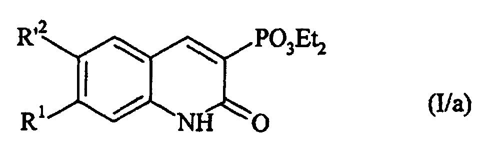 dans laquelle R¹ et R'² sont tels que définis précédemment,
- ou composé de formule (VII) que l'ou soumet à l'action du 2,5-diméthoxytétrahydrofurane pour conduire au composé de formule (I/b), cas particulier des composés de formule (I) 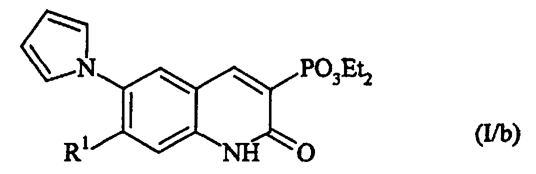
dans laquelle R¹ est tel que défini précédemment,
les composés de formule (I/a) et (I/b) pouvant être partiellement ou totalement déprotégés en présence de bromure de triméthylsilane par exemple pour conduire au composé de formule (I/c), cas particulier des composés de formule (I) : 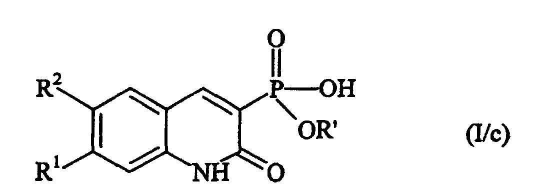 dans laquelle R¹ et R² sont tels que définis précédemment et R' représente un atome d'hydrogène ou un groupement éthyle,
sur lequel on peut condenser un composé de formule (X) :

R"-Cl (X)

dans laquelle R" représente un groupement alkyle, aryle, arylalkyle ou un groupement 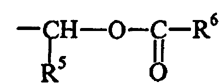 (où R⁵ et R⁶ sont tels que définis précédemment),
pour obtenir le composé de formule (I/d), cas particulier des composés de formule (I) : 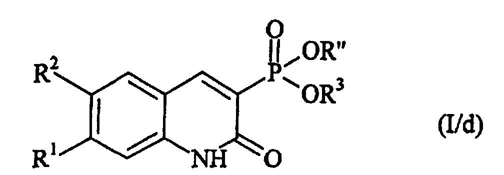 dans laquelle R¹, R², R³ et R" sont tels que définis précédemment,

les composés de formule (I/a) à (I/d) formant l'ensemble des composés de formule (I), et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

Les composés de l'invention présentent des propriétés pharmacologiques tout à fait intéressantes puisqu'ils sont de puissants inhibiteurs du récepteur AMPA, de surcroît sélectifs puisqu'ils ne touchent pas le récepteur NMDA et sont donc dépourvus de tous les effets secondaires décrits pour les antagonistes NMDA. L'utilisation de ces composés en tant qu'inhibiteurs des phénomènes pathologiques liés à l'hyperactivation des voies de neurotransmissions aux aminoacides excitateurs sera ainsi particulièrement appréciée dans les traitements aigus et surtout chroniques des maladies neurologiques et psychiques impliquant ces aminoacides comme les maladies dégénératives telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

La présente invention a également pour objet les compositions pharmaceutiques renfermant comme principe actif au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per
ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 50 mg et 10 g par 24 heures en 1 ou plusieurs prises.

Les exemples suivants illustrent l'invention et ne la limitent en aucune façon.

### EXEMPLE 1 : Acide (7-chloro-2-oxo-6-phényl-1,2-dihydro-3-quinolinyl) phosphonique diéthyl ester

### Stade A : Acide [(5-chloro -2 -formyl-phénylcarbamoyl)methyl]phosphonique diéthyl ester

A une solution de 2-amino-4-chloro-benzaldéhyde (6,18 g, 39,7 mmol) dans 170 ml de toluène anhydre on rajoute la pyridine (3,7 ml, 45,7 mmol) puis goutte à goutte une solution d'acide chlorocarbonylméthyl-phosphonique diéthyl ester (9,8 g, 45,7 mmol) dans 15 ml de toluène anhydre en maintenant la réaction à une température inférieure à 30 °C. A la fin de l'addition, on agite 1 heure à température ambiante. La réaction est lavée plusieurs fois à l'eau puis avec une solution HCl 1N, puis de nouveau à l'eau. On termine par un lavage avec une solution aqueuse de NaCl saturée. La phase organique est séchée sur MgSO₄, on filtre, évapore et on obtient le produit brut attendu sous forme d'une huile orange. Le produit brut est engagé dans l'étape suivante.

### Stade B : Acide (7-chloro-2-oxo-1,2-dihydro-3-quinolinyl)phosphonique diéthyl ester

Dans un ballon surmonté d'un Dean-Stark on porte au reflux sous forte agitation pendant 4 heures la totalité du composé obtenu au stade A en solution dans 300 ml de toluène et 0,3 ml de pipéridine. On laisse cristalliser à température ambiante et on filtre le solide jaune pale obtenu correspondant au produit du titre.
*Point de fusion : 210-213°C*

| *Microanalyse élémentaire :* | | | | |
|---|---|---|---|---|
| | *C%* | *H%* | *N%* | *Cl%* |
| *calculé* | *49,46* | *4,79* | *4,44* | *11,23* |
| *trouvé* | *49,77* | *4,78* | *4,46* | *11,63* |

### Stade C : Acide (7-chloro-6-nitro-2-oxo-1,2-dihydro-3-quinolinyl)phosphonique diéthyl ester

A une solution de 55 ml d'acide sulfurique 96 % refroidit dans un bain de glace on ajoute goutte à goutte 55 ml d'acide nitrique puis portion par portion le composé obtenu au stade B (14,7 g, 46,6 mmol) en maintenant la température inférieure ou égale à 5 °C. A la fin de l'addition on poursuit l'agitation 15 minutes puis on retire le bain de glace et la réaction est amenée à température ambiante en 1 heure 30 environ. La solution est versée sur de la glace et le précipité est agité jusqu'à obtenir un solide filtrable. On filtre, on lave à l'eau jusqu'à neutralité puis on sèche sous vide. Le solide est repris en suspension dans 210 ml d'éthanol au reflux ; on laisse refroidir et filtre pour obtenir après séchage le composé du titre.
*Point de fusion : 258-262°C*

| *Microanalyse élémentaire :* | | | | |
|---|---|---|---|---|
| | *C%* | *H%* | *N%* | *Cl%* |
| *calculé* | *43,29* | *3,91* | *7,77* | *9,83* |
| *trouvé* | *43,33* | *4,06* | *7,60* | *9,70* |

### Stade D : Acide (6-amino-7-chloro-2-oxo-1,2-dihydro-3-quinolinyl)phosphonique diéthyl ester

Une suspension du composé obtenu au stade C (7,0 g, 19,4 mmol), de Fer en poudre (10,8 g, 194 mmol) et de chlorure d'ammonium (10,4 g, 194 mmol) est agitée au reflux pendant 1 heure dans un mélange de 270 ml de méthanol et 90 ml d'eau. La suspension est filtrée à chaud sur célite et on rince plusieurs fois la célite avec du méthanol. Le filtrat est évaporé à sec et le résidu est repris en suspension dans de l'eau. On filtre le solide qui est rincé avec de l'eau et séché pour conduire au produit du titre sous forme de cristaux oranges.
*Point de fusion : 255-260°C*

| *Microanalyse élémentaire :* | | | |
|---|---|---|---|
| | *C%* | *H%* | *N%* |
| *calculé* | *47,22* | *4,88* | *8,47* |
| *trouvé* | *47,06* | *4,99* | *8,08* |

### Stade E : 7-Chloro-3 -(diéthoxyphosphoryl)-2-oxo-1,2-dihydro -6-quinoline-diazonium tetrafluoroborate

A un mélange de 4 ml de HBF₄ 48% aqueux et 4 ml d'eau on ajoute en petites portions le composé obtenu au stade D (2,0 g, 6,05 mmol). La suspension est agitée 10 minutes à température ambiante puis à 5°C dans un bain de glace. Parallèlement on prépare une solution de NaNO₂ (416 mg, 6,05 mmol) dans 1 ml d'eau. Cette solution est rajoutée à la suspension d'amine préparée plus haut. Le milieu réactionnel passe rapidement en solution puis on observe une prise en masse de la réaction. On rajoute un peu d'eau jusqu'à rendre possible l'agitation que l'on poursuit 10 minutes. Le précipité épais est ensuite filtré sur fritté et rincé avec un minimum d'eau. Après séchage sous vide en présence de P₂O₅ on obtient le diazonium tetrafluoroborate du titre sous forme d'une poudre beige.
*Point de fusion : 184-188°C*

### Stade F : Acide (7 -chloro-2-oxo-6-phényl-1,2-dihydro -3 -quinolinyl)phosphonique diéthyl ester

On agite pendant une nuit 500 mg (1,16 mmol) du composé obtenu au stade E, 170 mg (1,39 mmol) d'acide phénylboronique et 26 mg (0,11 mmol) de Pd(OAc)₂ dans un mélange de 40 ml de dioxanne et 40 ml d'éthanol. La solution noire est évaporée à sec et le résidu est repris dans environ 100 ml d'acétate d'éthyle. On filtre un insoluble noir. Le filtrat est lavé avec de l'eau puis avec une solution saturée de NaCl. On sèche sur MgSO₄, filtre, évapore à sec, cristallise le résidu dans de l'éther éthylique et filtre les cristaux pour obtenir après séchage le composé du titre.
*Point de fusion : 194°C*

| *Microanalyse élémentaire :* | | | | |
|---|---|---|---|---|
| | *C%* | *H%* | *N%* | *Cl%* |
| *calculé* | *58,25* | *4,89* | *3,58* | *9,05* |
| *trouvé* | *58,09* | *4,97* | *3,52* | *9,62* |

### EXEMPLE 2 : Acide (7-chloro-2-oxo-6-phényl-1,2-dihydro-3-quinolinyl) phosphonique

Dans 10 ml d'acétonitrile anhydre on met en suspension le composé obtenu dans l'Exemple 1 (130 mg, 0,33 mmol), on ajoute 0,435 ml (3,3 mmol) de bromotriméthylsilane et on agite à reflux 1 heure. On évapore à sec. Le résidu est séché sous vide et on le dissout dans du méthanol. On agite la solution pendant 20 minutes, évapore à sec, reprend dans de l'acétonitrile, triture jusqu'à obtention d'un précipité homogène. Après filtration du précipité blanc et rinçage avec un peu d'acétonitrile puis d'éther on obtient le composé du titre.
*Point de fusion :* > *260°C*

| *Microanalyse élémentaire :* | | | | |
|---|---|---|---|---|
| | *C%* | *H%* | *N%* | *Cl%* |
| *calculé* | *53,67* | *3,30* | *4,17* | *10,56* |
| *trouvé* | *53,30* | *3,31* | *4,14* | *11,12* |

Les Exemples 3 à 22 sont obtenus en procédant comme dans les Exemples 1 et 2 à partir des substrats appropriés.

### EXEMPLE 3 : Acide [7-chloro-6-(4-nitrophényl)-2-oxo-1,2-dihydro-3-quinolinyl] phosphonique diéthyl ester

On procède comme dans l'Exemple 1 en remplaçant dans le stade F l'acide phénylboronique par l'acide (4-nitrophényl)boronique, pinacolique ester.
*Point de fusion : 256-259°C*

| *Microanalyse élémentaire :* | | | | |
|---|---|---|---|---|
| | *C%* | *H%* | *N%* | *Cl%* |
| *calculé* | *52,25* | *4,15* | *6,41* | *8,12* |
| *trouvé* | *52,36* | *4,18* | 6,38 | *8,53* |

### EXEMPLE 4 : Acide [7-chloro-6-(4-nitrophényl)-2-oxo-1,2-dihydro-3-quinolinyl] phosphonique

On procède comme dans l'Exemple 2 à partir du composé obtenu dans l'Exemple 3.
*Point de fusion : 228°C*

| *Microanalyse élémentaire :* | | | | |
|---|---|---|---|---|
| | *C%* | *H%* | *N%* | *Cl%* |
| *calculé* | *47,33* | *2,65* | *7,36* | *9,31* |
| *trouvé* | *47,86* | *2,51* | *7,31* | *9,59* |

### EXEMPLE 5 : Acide [7-chloro-6-(3-nitrophényl)-2-oxo-1,2-dihydro-3-quinolinyl] phosphonique diéthyl ester

On procède comme dans l'Exemple 1 en remplaçant dans le stade F l'acide phényl-boronique par l'acide (3-nitrophényl)boronique.
*Point de fusion : 210-212°C*

| *Microanalyse élémentaire :* | | | | |
|---|---|---|---|---|
| | *C%* | *H%* | *N%* | *Cl%* |
| *calculé* | *52,25* | *4,15* | *6,41* | *8,12* |
| *trouvé* | *52,24* | *4,19* | *6,21* | *9,66* |

### EXEMPLE 6 : Acide [7-chloro-6-(3-nitrophényl)-2-oxo-1,2-dihydro-3-quinolinyl] phosphonique

On procède comme dans l'Exemple 2 à partir du composé obtenu dans l'Exemple 5.
*Point de fusion :* > *310°C*

| *Microanalyse élémentaire :* | | | | |
|---|---|---|---|---|
| | *C%* | *H%* | *N%* | *Cl%* |
| *calculé* | *47,33* | *2,65* | *7,36* | *9,31* |
| *trouvé* | *47,97* | *2,66* | *7,31* | *10,09* |

### EXEMPLE 7 : Acide (7-chloro-2-oxo-6-(3-thiényl)-1,2-dihydro-3-quinolinyl) phosphonique diéthyl ester

On procède comme dans l'Exemple 1 en remplaçant dans le stade F l'acide phényl-boronique par l'acide (3-thiényl)boronique.
*Point de fusion :* 170-172°C

| *Microanalyse élémentaire :* | | | | | |
|---|---|---|---|---|---|
| | *C%* | *H%* | *N%* | *S%* | *Cl%* |
| *calculé* | *51,33* | *4,31* | *3,52* | *8,06* | *8,91* |
| *trouvé* | *50,93* | *4,21* | *3,69* | *7,32* | *9,56* |

### EXEMPLE 8 : Acide (7-chloro-2-oxo-6-(3-thiényl)-1,2-dihydro-3-quinolinyl) phosphonique

On procède comme dans l'Exemple 2 à partir du composé obtenu dans l'Exemple 7.
*Point de fusion : 245-248°C*

| *Microanalyse élémentaire :* | | | | | |
|---|---|---|---|---|---|
| | *C%* | *H%* | *N%* | *S%* | *Cl%* |
| *calculé* | *45,69* | *2,65* | *4,10* | *9,38* | *10,38* |
| *trouvé* | *45,35* | *2,75* | *4,38* | *9,00* | *11,00* |

### EXEMPLE 9 : Acide (7-chloro-6-(1-naphtyl)-2-oxo-1,2-dihydro-3-quinolinyl) phosphonique diéthyl ester

On procède comme dans l'Exemple 1 en remplaçant dans le stade F l'acide phényl-boronique par l'acide (1-naphtyl)boronique.
*Point de fusion : 224-228°C*

### EXEMPLE 10 : Acide (7-chloro-6-(1-naphtyl)-2-oxo-1,2-dihydro-3-quinolinyl) phosphonique

On procède comme dans l'Exemple 2 à partir du composé obtenu dans l'Exemple 9.
*Point de fusion :* > *260°C*

| *Microanalyse élémentaire :* | | | | |
|---|---|---|---|---|
| | *C%* | *H%* | *N%* | *Cl%* |
| *calculé* | *59,16* | *3,40* | *3,63* | *9,19* |
| *trouvé* | *58,78* | *3,45* | *3,69* | *8,88* |

### EXEMPLE 11 : Acide {7-chloro-2-oxo-6-[4-(trifluorométhyl)phényl]-1,2-dihydro-3-quinolinyl}phosphonique diéthyl ester

On procède comme dans l'Exemple 1 en remplaçant dans le stade F l'acide phényl-boronique par l'acide [4-(tnfluorométhyl)phényl]boronique.
*Point de fusion : 245-249°C*

| *Microanalyse élémentaire :* | | | |
|---|---|---|---|
| | *C%* | *H%* | *N%* |
| *calculé* | *52,25* | *3,95* | *3,05* |
| *trouvé* | *52,30* | *4,19* | *3,11* |

### EXEMPLE 12 : Acide {7-chloro-2-oxo-6-[4-(trifluorométhyl)phényl]-1,2-dihydro-3-quinolinyl}phosphonique

On procède comme dans l'Exemple 2 à partir du composé obtenu dans l'Exemple 11.
*Point de fusion : 245°C*

| *Microanalyse élémentaire :* | | | | |
|---|---|---|---|---|
| | *C%* | *H%* | *N%* | *Cl%* |
| *calculé* | *47,61* | *2,50* | *3,47* | *8,78* |
| *trouvé* | *47,50* | *2,62* | *3,49* | *8,98* |

### EXEMPLE 13 : Acide [7-chloro-6-(2-naphtyl)-2-oxo-1,2-dihydro-3-quinolinyl] phosphonique diéthyl ester

On procède comme dans l'Exemple 1 en remplaçant dans le stade F l'acide phényl-boronique par l'acide (2-naphtyl)boronique.
*Point de fusion : 243-275°C*

| *Microanalyse élémentaire :* | | | |
|---|---|---|---|
| | *C%* | *H%* | *N%* |
| *calculé* | *62,52* | *4,79* | *3,17* |
| *trouvé* | *61,80* | *4,93* | *3,22* |

### EXEMPLE 14 : Acide [7-chloro-6-(2-naphtyl)-2-oxo-1,2-dihydro-3-quinolinyl] phosphonique

On procède comme dans l'Exemple 2 à partir du composé obtenu dans l'Exemple 13.
*Point de fusion :* > *300°C*

| *Microanalyse élémentaire :* | | | | |
|---|---|---|---|---|
| | *C%* | *H%* | *N%* | *Cl%* |
| *calculé* | *59,16* | *3,40* | *3,63* | *9,19* |
| *trouvé* | *59,02* | *3,46* | *3,61* | *9,54* |

### EXEMPLE 15 : Acide [7-chlore-6-(4-méthylphényl)-2-oxo-1,2-dihydro-3-quinolinyl] phosphonique diéthyl ester

On procède comme dans l'Exemple 1 en remplaçant dans le stade F l'acide phényl-boronique par l'acide (4-méthylphényl)boronique.
*Point de fusion : 259°C*

### EXEMPLE 16 : Acide [7-chloro-6-(4-méthylphényl)-2-oxo-1,2-dihydro-3-quinolinyl] phosphonique

On procède comme dans l'Exemple 2 à partir du composé obtenu dans l'Exemple 15.
*Point de fusion : > 300°C*

| *Microanalyse élémentaire :* | | | | |
|---|---|---|---|---|
| | *C%* | *H%* | *N%* | *Cl%* |
| *calculé* | *54,95* | *3,75* | *4,01* | *10,14* |
| *trouvé* | *54,55* | *3,73* | *4,27* | *10,68* |

### EXEMPLE 17 : Acide (6-[1,1'-biphényl]-4-yl-7-chloro-2-oxo-1,2-dihydro-3-quinolinyl)phosphonique diéthyl ester

On procède comme dans l'Exemple 1 en remplaçant dans le stade F l'acide phényl-boronique par l'acide (1,1'-biphényl)-4-yl boronique.
*Point de fusion : 280-281°C*

### EXEMPLE 18: Acide (6-[1,1'-biphényl]-4-yl-2-oxo-1,2-dihydro-3-quinolinyl)phosphonique

On procède comme dans l'Exemple 2 à partir du composé obtenu dans l'Exemple 17.
*Point de fusion :* > *260°C*

| *Microanalyse élémentaire :* | | | |
|---|---|---|---|
| | *C%* | *H%* | *N%* |
| *calculé* | *61,25* | *3,67* | *3,40* |
| *trouvé* | *61,17* | *3,87* | *3,49* |

### EXEMPLE 19 : Acide [7-chloro-6-(4-cyanophényl)-2-oxo-1,2-dihydro-3-quinolinyl] phosphonique diéthyl ester

On procède comme dans l'Exemple 1 en remplaçant dans le stade F l'acide phényl-boronique par l'acide (4-cyanophényl)boronique.
*Point de fusion : 273-275 °C*

| *Microanalyse élémentaire :* | | | | |
|---|---|---|---|---|
| | *C%* | *H%* | *N%* | *Cl%* |
| *calculé* | *57,63* | *4,35* | *6,72* | *8,51* |
| *trouvé* | *58,03* | *4,46* | *6,91* | *8,89* |

### EXEMPLE 20 : Acide [7-chloro-6-(4-cyanophényl)-2-oxo-1,2-dihydro-3-quinolinyl] phosphonique

On procède comme dans l'Exemple 2 à partir du composé obtenu dans l'Exemple 19.
*Point de fusion :* > *260°C*

| *Microanalyse élémentaire :* | | | | |
|---|---|---|---|---|
| | *C%* | *H%* | *N%* | *Cl%* |
| *calculé* | *53,28* | *2,79* | *7,77* | *9,83* |
| *trouvé* | *52,86* | *3,12* | *7,76* | *9,68* |

### EXEMPLE 21 : Acide [7-chloro-6-(4-fluorophényl)-2-oxo-1,2-dihydro-3-quinolinyl] phosphonique diéthyl ester

On procède comme dans l'Exemple 1 en remplaçant dans le stade F l'acide phényl-boronique par l'acide (4-fluorophényl)boronique.
*Point de fusion : 228°C*

| *Microanalyse élémentaire :* | | | |
|---|---|---|---|
| | *C%* | *H%* | *N%* |
| *calculé* | *55,69* | *4,43* | *3,42* |
| *trouvé* | *55,03* | *4,84* | *3,51* |

### EXEMPLE 22 : Acide [7-chloro-6-(4-fluorophényl)-2-oxo-1,2-dihydro-3-quinolinyl] phosphonique

On procède comme dans l'Exemple 2 à partir du composé obtenu dans l'Exemple 21.
*Point de fusion : 265°C*

| *Microanalyse élémentaire :* | | | | |
|---|---|---|---|---|
| | *C%* | *H%* | *N%* | *Cl%* |
| *calculé* | *50,94* | *2,85* | *3,96* | *10,02* |
| *trouvé* | *50,86* | *3,03* | *4,18* | *10,40* |

### EXEMPLE 23 : Acide [2-oxo-6-phényl-7-(trifluorométhyl)-1,2-dihydro-3-quinolinyl]phosphonique diéthyl ester

On procède comme dans l'Exemple 1 en remplaçant au stade A le 2-amino-4-chloro benzaldéhyde par le 2-amino-4-trifluorométhylbenzaldéhyde, et en réalisant l'étape de réduction au stade D par le couple Pd-C/formiate d'ammonium au lieu du couple Fe/NH₄Cl en milieu hydroalcoolique.

### Stade A : Acide [(5-trifluorométhyl-2-formyl-phénylcarbamoyl)méthyl] phosphonique diéthyl ester

*Point de fusion : 62-64°C*

| *Microanalyse élémentaire :* | | | |
|---|---|---|---|
| | *C%* | *H%* | *N%* |
| *calculé* | *45,79* | *4,67* | *3,81* |
| *trouvé* | *45,89* | *4,66* | *3,76* |

### Stade B : Acide (7-trifluorométhyl-2-oxo-1,2-dihydro-3-quinolinyl)phosphonique diéthyl ester

*Point de fusion : 151°C*

| *Microanalyse élémentaire :* | | | |
|---|---|---|---|
| | *C%* | *H%* | *N%* |
| *calculé* | *48,15* | *4,33* | *4,01* |
| *trouvé* | *48,19* | *4,32* | *3,92* |

### Stade C : Acide (7-trifluorométhyl-6-nitro-2-oxo-1,2-dihydro-3-quinolinyl) phosphonique diéthyl ester

*Point de fusion : 209-215°C*

| *Microanalyse élémentaire :* | | | |
|---|---|---|---|
| | *C%* | *H%* | *N%* |
| *calculé* | *42,65* | *3,58* | *7,11* |
| *trouvé* | *42,86* | *3,58* | *6,78* |

### Stade D : Acide (6-amino-7-trifluorométhyl-2-oxo-1,2-dihydro-3 -quinolinyl) phosphonique diéthyl ester

On agite à reflux pendant 1 heure un mélange de 490 mg (1,24 mmol) du composé obtenu au stade D, 650 mg (12,4 mmol) de formiate d'ammonium et 120 mg de Pd/C 10 % dans 50 ml d'éthanol. Le catalyseur est filtré sur membrane, le filtrat est évaporé à sec et le résidu est repris dans l'eau. On filtre la suspension, rince à l'eau, essore et sèche sous vide pour obtenir le produit du titre sous la forme d'un solide jaune.
*Point de fusion : 240-244°C*

| *Microanalyse élémentaire :* | | | |
|---|---|---|---|
| | *C%* | *H%* | *N%* |
| *calculé* | *46,16* | *4,43* | *7,69* |
| *trouvé* | *46,26* | *4,37* | *7,62* |

### Stade E : 7-Trifluorométhyl-3-(diéthoxyphosphoryl)-2-oxo-1,2-dihydro-6-quinoline-diazonium tetrafluoroborate

### Stade F : Acide [2-oxo-6-phényl-7 -(trifluorométhyl)-1,2-dihydro-3-quinolinyl] phosphonique diéthyl ester

*Point de fusion : 211-214°C*

### EXEMPLE 24 : Acide (2-oxo-6-phényl-7-trifluorométhyl-1,2-dihydro-3-quinolinyl] phosphonique

On procède comme dans l'Exemple 2 à partir du composé obtenu dans l'Exemple 23.
*Point de fusion :* > *300°C*

| *Microanalyse élémentaire :* | | | |
|---|---|---|---|
| | *C%* | *H%* | *N%* |
| *calculé* | *52,05* | *3,00* | *3,79* |
| *trouvé* | *51,69* | *2,93* | *3,76* |

### EXEMPLE 25 : Acide {7-fluoro-2-oxo-6-[4-(trifluorométhyl)phényl]-1,2-dihydro-3-quinolinyl}phosphonique diéthyl ester

On procède comme dans l'Exemple 1 en remplaçant au stade A le 2-amino-4-chloro-benzaldéhyde par le 2-amino-4-fluoro-benzaldéhyde.

### Stade A : Acide [(5-fluoro-2-formyl-phénylcarbamoyl)méthyl]phosphonique diéthyl ester

Produit non isolé (huile) engagé tel quel dans le stade B.

### Stade B : Acide (7-fluoro-2-oxo-1,2-dihydro-3-quinolinyl)phosphonique diéthyl ester

*Point de fusion : 230-233°C*

| *Microanalyse élémentaire :* | | | |
|---|---|---|---|
| | *C%* | *H%* | *N%* |
| *calculé* | *52,18* | *5,05* | *4,68* |
| *trouvé* | *52,22* | *5,23* | *4,62* |

### Stade C : Acide (7-fluoro-6-nitro-2-oxo-1,2-dihydro-3-quinolinyl )phosphonique diéthyl ester

*Point de fusion : 259-262°C*

| *Microanalyse élémentaire :* | | | |
|---|---|---|---|
| | *C%* | *H%* | *N%* |
| *calculé* | *45,36* | *4,10* | *8,14* |
| *trouvé* | *45,40* | *4,28* | *8,06* |

### Stade D : Acide (6-amino-7-fluoro-2-oxo-1,2-dihydro-3-quinolinyl)phosphonique diéthyl ester

*Point de fusion : 253-257°C*

| *Microanalyse élémentaire :* | | | |
|---|---|---|---|
| | *C%* | *H%* | *N%* |
| *calculé* | *49,69* | *5,13* | *8,91* |
| *trouvé* | *49,52* | *5,28* | *8,70* |

### Stade E : 7-Fluoro-3-(diéthoxyphosphoryl)-2-oxo-1,2-dihydro-6-quinolinediazonium tetrafluoroborate

*Point de fusion : 127-131°C*

### Stade F : Acide {7-fluoro-2-oxo-6-[4-(trifluorométhyl)phényl]-1,2-dihydro -3-quinolinyl}phosphonique diéthyl ester

*Point de fusion : 258-260°C*

| *Microanalyse élémentaire :* | | | |
|---|---|---|---|
| | *C%* | *H%* | *N%* |
| *calculé* | *54,19* | *4,09* | *3,16* |
| *trouvé* | *54,01* | *4,45* | *3,35* |

### EXEMPLE 26 : Acide [7-fluoro-6-(4-trifluorométhyl-phényl)-2-oxo-1,2-dihydroquinolin-3-yl]phosphonique

On procède comme dans l'Exemple 1 à partir du composé obtenu dans l'Exemple 25.
*Point de fusion : 267°C*

| *Microanalyse élémentaire :* | | | |
|---|---|---|---|
| | *C%* | *H%* | *N%* |
| *calculé* | *49,63* | *2,60* | *3,62* |
| *trouvé* | *50,33* | *2,57* | *3,63* |

### EXEMPLE 27 : Acide [7-chloro-2-oxo-6-(1H-pyrrol-1-yl)-1,2-dihydro-3-quinolinyl]phosphonique diéthyl ester

Une suspension biphasique composée de 2,43 g (7,35 mmol) du composé obtenu au stade D de l'Exemple 1 et 1,33 ml (10,29 mmol) de 2,5-diméthoxy-tétrahydrofuranne dans un mélange de 24 ml d'eau distillée, 12 ml d'acide acétique et 36 ml de dichloro-1,2-éthane est agitée vigoureusement à 75°C pendant 60 minutes. En CCM (CH₂Cl₂/MeOH 9:1) on observe encore un peu de produit de départ. On rajoute 0,2 eq de 2,5-diméthoxy-tétrahydrofuranne (2,06 mmol, 0,26 ml) et poursuit le chauffage 30 minutes. On laisse revenir à température ambiante et on extrait avec du CH₂Cl₂. La phase organique est lavée 4 fois à l'eau puis avec une solution de NaCl saturée. On sèche sur MgSO₄, évapore et chromatographie l'huile obtenue sur 200 g de silice Merck 7734 en éluant avec un mélange CH₂Cl₂/MeOH 95:5. On obtient une huile correspondant au produit du titre qui est cristallisée dans de l'éther isopropylique.
*Point de fusion : 193-196°C*

| *Microanalyse élémentaire :* | | | | |
|---|---|---|---|---|
| | *C%* | *H%* | *N%* | *Cl%* |
| *calculé* | *53,63* | *4,76* | *7,36* | *9,31* |
| *trouvé* | *53,92* | *4,89* | *7,28* | *9,55* |

### EXEMPLE 28 : Acide [7-chloro-2-oxo-6-(1H-pyrrol-1-yl)-1,2-dihydro-3-quinolinyl] phosphonique

A une solution du composé obtenu dans l'Exemple 27 (1,62 g, 4,25 mmol) dans 30 ml de CH₂Cl₂, on ajoute 3,37 ml (25,5 mmol) de bromotriméthylsilane et on agite 4 heures 30 la solution à température ambiante. On évapore à sec, reprend le résidu dans du méthanol. Après solubilisation totale on observe une précipitation que l'on agite 10 minutes à température ambiante. On filtre, rince avec un peu de méthanol puis d'éther pour obtenir le produit du titre.
*Point de fusion : 195-200°C*

| *Microanalyse élémentaire :* | | | | |
|---|---|---|---|---|
| | *C %* | *H%* | *N%* | *Cl%* |
| *calculé* | *48,09* | *3,10* | *8,63* | *10,92* |
| *trouvé* | *48,37* | *3,39* | *7,85* | *10,79* |

### EXEMPLE 29 : Acide [2-oxo-6-(1H-pyrrol-1-yl)-7-trifluorométhyl-1,2-dihydro-3-quinolinyl]phosphonique diéthyl ester

On procède comme dans l'Exemple 27 à partir du composé obtenu au stade D de l'Exemple 23.
*Point de fusion : 229-234°C*

| *Microanalyse élémentaire :* | | | |
|---|---|---|---|
| | *C%* | *H%* | *N%* |
| *calculé* | *52,18* | *4,38* | *6,76* |
| *trouvé* | *52,23* | *4,42* | *6,73* |

### EXEMPLE 30 : Acide [2-oxo-6-(1H-pyrrol-1-yl)-7-trifluorométhyl-1,2-dihydro-3-quinolinyl]phosphonique

On chauffe au reflux de l'acétonitrile (3 ml) pendant 1 heure 30 une suspension de 235 mg (0,567 mmol) du composé obtenu dans l'Exemple 29 et 0,75 ml (5,67 mmoles) de bromotriméthylsilane. On évapore à sec, reprend dans du méthanol, agite la solution 30 minutes puis évapore à sec de nouveau. Le résidu est trituré dans l'acétonitrile et on filtre le précipité correspondant au produit du titre.
*Point de fusion : 211-213°C*

| *Microanalyse élémentaire :* | | | |
|---|---|---|---|
| | *C%* | *H%* | *N%* |
| *calculé* | *46,94* | *2,81* | *7,82* |
| *trouvé* | *46,64* | *3,25* | *7,37* |

### EXEMPLE 31 : Acide 6-[4-(aminosulfonyl)phényl]-7-chloro-2-oxo-1,2-dihydro-3-quinolinyl phosphonique diéthyl ester

On procède comme au stade F de l'exemple 1 en remplaçant l'acide phényl-boronique par l'acide 4-sulfamoyl-phényl boronique pinacolester.
*Point de fusion : 292-295°C*

| *Microanalyse élémentaire :* | | | | | |
|---|---|---|---|---|---|
| | *C%* | *H%* | *N%* | *S%* | *Cl%* |
| *calculé* | *48,47* | *4,28* | *5,95* | *6,81* | *7,53* |
| *trouvé* | *48,80* | *4,44* | *6,50* | *6,58* | *7,40* |

### EXEMPLE 32 : Acide 6-[4-(aminosulfonyl)phényl]-7-chloro-2-oxo-1,2-dihydro-3-quinolinyl phosphonique

On procède comme dans l'exemple 2 à partir du composé obtenu dans l'exemple 31.
*Point de fusion :* > *300°C*

| *Microanalyse élémentaire :* | | | | | |
|---|---|---|---|---|---|
| | *C%* | *H%* | *N%* | *S%* | *Cl%* |
| *calculé* | *43,44* | *2,92* | *6,75* | *7,73* | *8,55* |
| *trouvé* | *43,12* | *2,84* | *6,61* | *7,50* | *8,38* |

### ETUDE PHARMACOLOGIQUE

### EXEMPLE A : Inhibition du courant induit par une application de (R,S)-AMPA (10 µM) sur des ovocytes de Xenope injectés avec des ARNm de cortex de rat

Des ovocytes de Xenopes sont injectés avec 50 ng d'ARNm poly (A+) isolés de cortex cérébral de rat et incubés 2 à 3 jours à 18°C pour en permettre l'expression protéique. Les courants entrants induits par une application de (R,S)-AMPA (10 µM) sont mesurés dans un milieu de composition : NaCl (82,5 mM), KCl ( 2,5 mM), CaCl₂ (1 mM), MgCl₂ (1 mM), NaH₂PO₄ (1 mM), HEPES (5 mM), pH 7,4 par la méthode du voltage-clamp à 2 électrodes (potentiel = - 60 mV). Les produits de la présente invention sont appliqués de façon concentration dépendante 30 secondes avant et durant l'application de l'agoniste (R,S)-AMPA.

Leur capacité à inhiber le courant induit par le (R,S)-AMPA est déterminée par les valeurs d'IC50 (µM), représentant les concentrations inhibitant de 50 % le courant induit par une application de (R,S)-AMPA (10 µM).
Les composés de l'invention montrent d'excellentes propriétés inhibitrices avec des IC50 (µM) de l'ordre de 1.

### EXEMPLE B : Test des convulsions audiogènes chez la souris DBA/2

Chez la souris DBA/2 immature, des crises convulsives peuvent être déclenchées en soumettant cet animal à une stimulation sonore de haute intensité et de haute fréquence.
Les antagonistes des récepteurs au glutamate de type AMPA antagonisent ce type de convulsions de façon dose-dépendante (Chapman et al., Epilepsy Res., 1991, 9, 92-96). Ce test est utilisé pour étudier les effets anti-convulsivants des composés de la présente invention. Brièvement, des souris DBA/2 immatures (21-28 jours) sont exposées pendant 60 secondes à un bruit de 105 Db et de 18 KH_{z}. Ceci entraîne l'apparition de convulsions cloniques. Les produits sous étude et le solvant sont administrés par voie i.p. 30 minutes avant le début du test sous un volume de 0,1 ml/10 g. Les ED50 (doses inhibant de 50 % l'incidence des convulsions) est déterminée pour chaque composé en utilisant la méthode de Litchfield et Wicoxon (J. Pharmacol. Exp. Ther., 1949, 96, 99-113).
Les composés de l'invention montrent une excellente capacité à inhiber les convulsions avec des ED50 de l'ordre de 10 mg/kg ip.

### EXEMPLE C : Composition pharmaceutique

| | |
|---|---|
| 1000 comprimés dosés à 5 mg de l'acide {7-chloro-2-oxo-6-[4-(trifluorométhyl)phényl]-1,2-dihydro-3-quinolinyl}phosphonique (Exemple 12) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 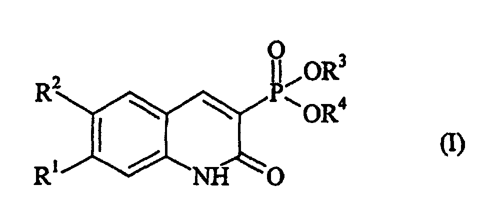 dans laquelle :
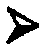 R¹ représente un atome d'halogène ou un groupement trifluorométhyle,
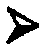 R² représente un groupement aryle ou hétéroaryle,
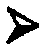 R³ et R⁴, identiques ou différents, représentent un atome d'hydrogène ou un groupement alkyle, cycloalkyle, aryle, arylalkyle ou un groupement 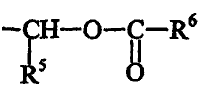 (dans lequel R⁵ et R⁶, identiques ou différents, représentent un atome d'hydrogène ou un groupement alkyle, cycloalkyle ou aryle),
étant entendu que :
- par alkyle on entend un groupement alkyle contenant 1 à 6 atomes de carbone, linéaire ou ramifié,
- par cycloalkyle on entend un groupement alkyle cyclique contenant 3 à 8 atomes de carbone,
- par aryle on entend les groupements phényle, naphtyle ou biphényle, ces groupements pouvant être non substitués ou substitués par 1 à 3 groupements choisis parmi alkyle, cycloalkyle, alkoxy, polyhalogénoalkyle, cyano, nitro, amino, alkylamino, dialkylamino, SO₂NR⁷R⁸ (où R⁷ et R⁸, identiques ou différents, représentent un atome d'hydrogène ou un groupement alkyle, cycloalkyle ou aryle) ou atomes d'halogène,
- par hétéroaryle on entend tout groupement aromatique mono ou bicyclique contenant 5 à 10 sommets et contenant 1 à 3 hétéroatomes choisis parmi oxygène, azote et soufre, l'hétéroaryle pouvant être rattaché au noyau benzénique qui le porte par un atome de carbone ou par un atome d'azote lorsqu'il en possède un et pouvant être non substitué ou substitué par 1 à 3 groupements choisis parmi alkyle, cycloalkyle, alkoxy, polyhalogénoalkyle, cyano, nitro, amino, alkylamino, dialkylamino, SO₂NR⁷R⁸ (où R⁷ et R⁸ sont tels que définis précédemment), ou atomes d'halogène,
leurs énantiomères et diastéréoisomères ainsi que leurs sels d'addition à un acide ou à une base pharmaceutiquement acceptable.

2. Composés de formule (I) selon la revendication 1 pour lesquels R¹ représente un atome de chlore ou un groupement CF₃, leurs isomères ainsi que leurs sels d'addition à un acide ou à une base pharmaceutiquement acceptables.

3. Composés de formule (I) selon la revendication 1 pour lesquels R² représente un groupement naphtyle, phényle ou biphényle non substitués ou substitués, leurs isomères ainsi que leurs sels d'addition à un acide ou à une base pharmaceutiquement acceptables.

4. Composés de formule (I) selon la revendication 1 pour lesquels R² représente un groupement pyrrole ou thiophène, leurs isomères ainsi que leurs sels d'addition à un acide ou à une base pharmaceutiquement acceptables.

5. Composés de formule (I) selon la revendication 1 pour lesquels R³ et R⁴ représentent simultanément un atome d'hydrogène, leurs isomères ainsi que leurs sels d'addition à un acide ou à une base pharmaceutiquement acceptables.

6. Composés de formule (I) selon la revendication 1 qui sont l'acide {7-chloro-2-oxo-6-[4-(trifluorométhyl)phényl]-1,2-dihydro-3-quinolinyl}phosphonique et l'acide [7-chloro-6-(4-méthylphényl)-2-oxo-1,2-dihydro-3-quinolinyl]phosphonique, leurs isomères ainsi que leurs sels d'addition à une base pharmaceutiquement acceptable.

7. Procédé de préparation des composés de formule (I) selon la revendication 1 **caractérisé en ce que** l'on utilise comme produit de départ le composé de formule (II) : 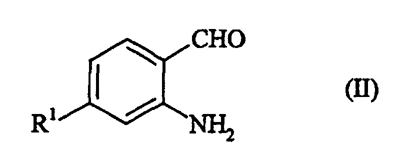 dans laquelle R¹ est tel que défini dans la formule (I),
sur lequel on condense en présence de base comme la pyridine par exemple un composé de formule (III) : 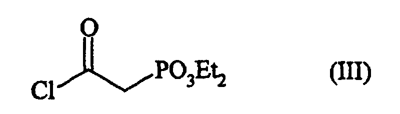 pour conduire au composé de formule (IV) : 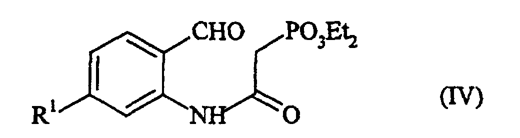 dans laquelle R¹ est tel que défini précédemment,
que l'on cyclise en présence d'une quantité catalytique de pipéridine pour obtenir le composé de formule (V): 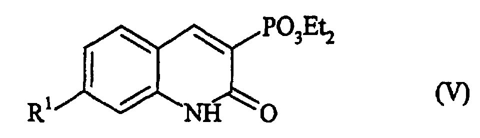 dans laquelle R¹ est défini comme précédemment,
que l'on soumet à un mélange d'acide nitrique et d'acide sulfurique pour conduire au composé de formule (VI) : 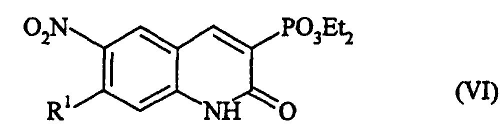 dans laquelle R¹ est tel que défini précédemment,
qui est réduit en utilisant du charbon palladié en présence d'hydrogène ou du Fer en milieu hydroalcoolique pour conduire au composé de formule (VII) : 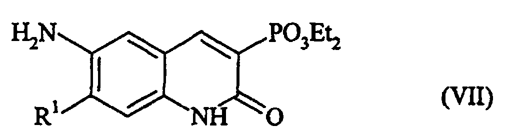 dans laquelle R¹ est défini comme précédemment,
- que l'on transforme par action de NaNO₂ et HBF₄ en sel fluoroborate de diazonium correspondant de formule (VIII) : 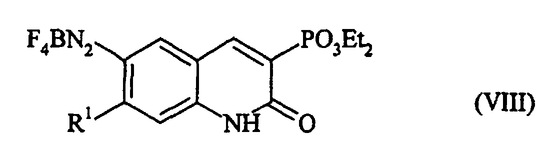
dans laquelle R¹ est tel que défini précédemment,
sur lequel on condense en présence de palladium un dérivé de l'acide boronique de formule (IX) : 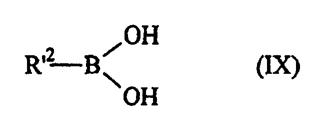 dans laquelle R'² représente un groupement aryle ou hétéroaryle tels que définis dans la formule (I), le groupement hétéroaryle étant rattaché à l'atome de bore par un atome de carbone,
pour conduire au composé de formule (I/a), cas particulier des composés de formule (I) : 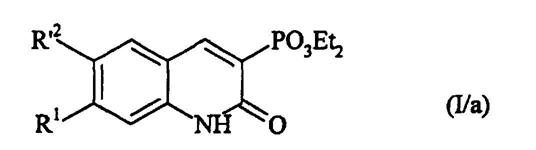 dans laquelle R¹ et R'² sont tels que définis précédemment,
- ou composé de formule (VII) que l'ou soumet à l'action du 2,5-diméthoxytétrahydrofurane pour conduire au composé de formule (I/b), cas particulier des composés de formule (I) : 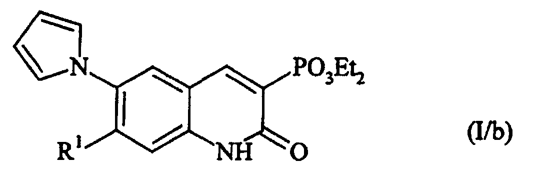
dans laquelle R¹ est tel que défini précédemment,
les composés de formule (I/a) et (I/b) pouvant être partiellement ou totalement déprotégés en présence de bromure de triméthylsilane par exemple pour conduire au composé de formule (I/c), cas particulier des composés de formule (I) : 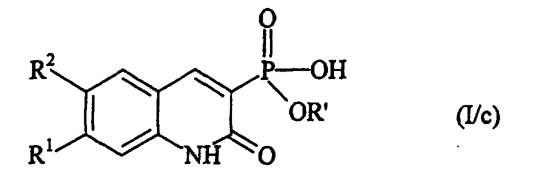 dans laquelle R¹ et R² sont tels que définis précédemment et R' représente un atome d'hydrogène ou un groupement éthyle,
sur lequel on peut condenser un composé de formule (X) :
R"-Cl (X)
dans laquelle R" représente un groupement alkyle, aryle, arylalkyle ou un groupement 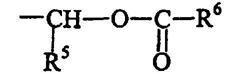 (où R⁵ et R⁶ sont tels que définis précédemment),
pour obtenir le composé de formule (I/d), cas particulier des composés de formule (I) : 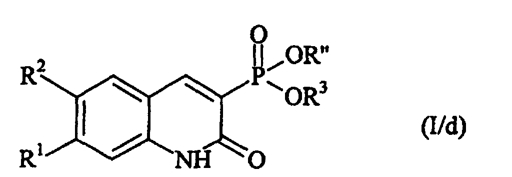 dans laquelle R¹, R², R³ et R" sont tels que définis précédemment,
les composés de formule (I/a) à (I/d) formant l'ensemble des composés de formule (I), et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

8. Compositions pharmaceutiques contenant comme principe actif au moins un composé de formule (I) selon l'une quelconque des revendications 1 à 6 ou un de ses sels d'addition à un acide ou à une base pharmaceutiquement acceptables, seul ou en combinaison avec un ou plusieurs excipients pharmaceutiquement acceptables.

9. Compositions pharmaceutiques selon la revendication 8 utiles pour la fabrication d'un médicament pour le traitement aigu et chronique des phénomènes pathologiques liés à l'hyperactivation des voies de neurotransmission aux aminoacides excitateurs comme l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington

## Patentansprüche

1. Verbindungen der Formel (I): 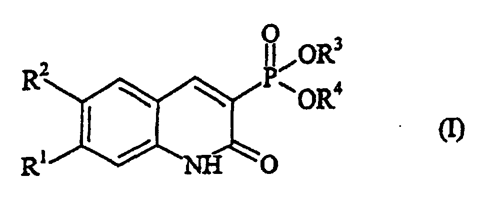 in der:
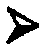 R¹ ein Halogenatom oder eine Trifluormethylgruppe.
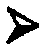 R² eine Aryl- oder Heteroarylgruppe.
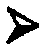 R³ und R⁴, die gleichartig oder verschieden sind. Wasserstoffatome oder Alkyl-, Cycloalkyl-, Aryl-, Arylalkylgruppen oder Gruppen 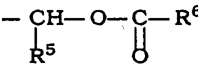 (in der R⁵ und R⁶, die gleichartig oder verschieden sind, Wasserstoffatome oder Alkyl-, Cycloalkyl- oder Arylgruppen darstellen) bedeuten,
wobei es sich versteht, daß:
- unter Alkyl eine geradkettige oder verzweigte Alkylgruppe zu verstehen ist, die 1 bis 6 Kohlenstoffatome enthält,
- unter Cycloalkyl eine cyclische Alkylgruppe zu verstehen ist, die 3 bis 8 Kohlenstoffatome enthält,
- unter Aryl Phenyl-, Naphthyl- oder Biphenylgruppen zu verstehen sind, welche Gruppen unsubstituiert oder durch 1 bis 3 Gruppen ausgewählt aus Alkyl, Cycloalkyl, Alkoxy, Polyhalogenoalkyl, Cyano, Nitro, Amino, Alkylamino, Dialkylamino, SO₂NR⁷R⁸ (worin R⁷ und R⁸, die gleichartig oder verschieden sind, Wasserstoffatome oder Alkyl-, Cycloalkyl- oder Arylgruppen darstellen) oder Halogenatomen substituiert sind,
- unter Heteroaryl man jede mono- oder bicyclische aromatische Gruppe versteht. die 5 bis 10 Kettenglieder aufweist und 1 bis 3 Heteroatome ausgewählt aus Sauerstoff, Stickstoff und Schwefel enthält, wobei Heteroaryl an einen Benzolkern gebunden sein kann, der diesen Rest über ein Kohlenstoffatom oder ein Stickstoffatom, falls er ein solches aufweist, trägt und nicht substituiert oder durch 1 bis 3 Gruppen ausgewählt aus Alkyl, Cycloalkyl, Alkoxy, Polyhalogenoalkyl, Cyano, Nitro, Amino, Alkylamino, Dialkylamino, SO₂NR⁷R⁸ (worin R⁷ und R⁸ die oben angegebenen Bedeutungen besitzen) oder Halogenatomen substituiert ist,
deren Enantiomere und Diastereoisomere sowie deren Additionssalze mit eine pharmazeutisch annehmbaren Säure oder Base.

2. Verbindungen der Formel (I) nach Anspruch 1, worin R¹ ein Chloratom ode eine Gruppe CF₃ darstellt, deren Isomere sowie deren Additionssalze mit eine pharmazeutisch annehmbaren Säure oder Base.

3. Verbindungen der Formel (I) nach Anspruch 1, worin R² eine nichtsubstitu ierte oder substituierte Naphthyl-, Phenyl- oder Biphenylgruppe bedeutet, deren Isomere sowie deren Additionssalze mit einer pharmazeutisch annehmbaren Säur oder Base.

4. Verbindungen der Formel (I) nach Anspruch 1, worin R² eine Pyrrol- ode: Thiophengruppe darstellt, deren Isomere sowie deren Additionssalze mit eine pharmazeutisch annehmbaren Säure oder Base.

5. Verbindungen der Formel (I) nach Anspruch 1, worin R³ und R⁴ gleichzeitig Wasserstoffatome bedeuten, deren Isomere sowie deren Additionssalze mit einen pharmazeutisch annehmbaren Säure oder Base.

6. Verbindungen der Formel (I) nach Anspruch 1, nämlich {7-Chlor-2-oxo-6-[4 (trifluormethyl)-phenyl]-1,2-dihydro-3-chinolinyl}-phosphonsäure und [7-Chlor-6-(4-methylphenyl)-2-oxo-1,2-dihydro-3-chinolinyll-phosphonsäure, deren Isomere sowie deren Additionssalze mit einer pharmazeutisch annehmbaren Säure odei Base.

7. Verfahren zur Herstellung der Verbindungen der Formel (I) nach Anspruch 1, **dadurch gekennzeichnet, daß** man als Ausgangsprodukt die Verbindung der Formel (II) verwendet: 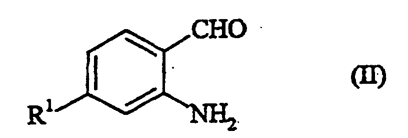 in der R¹ die bezüglich der Formel (I) angegebenen Bedeutungen besitzt, welche man in Gegenwart einer Base, wie beispielsweise Pyridin, mit einer Verbindung der Formel (III) kondensiert: 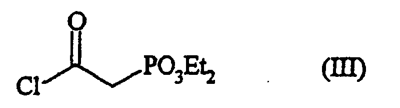 zur Bildung der Verbindung der Formel (IV): 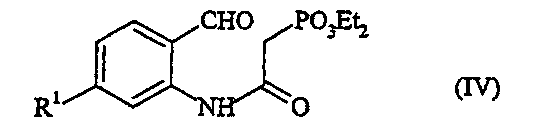 in der R¹ die oben angegebenen Bedeutungen besitzt,
welche man in Gegenwart einer katalytischen Menge Piperidins cyclisiert zur Bildung der Verbindung der Formel (V): 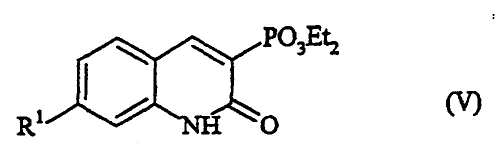 in der R¹ die oben angegebenen Bedeutungen besitzt,
welche man der Einwirkung einer Mischung aus Salpetersäure und Schwefelsäure unterwirft zur Bildung der Verbindung der Formel (VI): 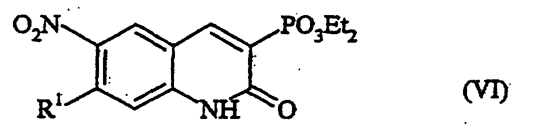 in der R¹ die oben angegebenen Bedeutungen besitzt,
welche man unter Verwendung von Palladium-auf-Kohlenstoff in Gegenwart von Wasserstoff oder von Eisen in einem wäßrig-alkoholischen Medium reduziert zur Bildung der Verbindung der Formel (VII): 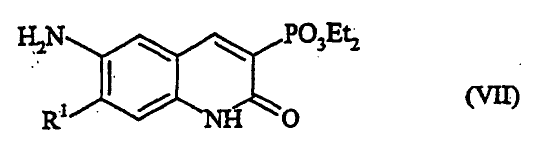 in der R¹ die oben angegebenen Bedeutungen besitzt,
- welche man durch Einwirkung von NaNO₂ und HBF₄ in das entsprechende Dia zoniumfluorborat der Formel (VIII) umwandelt: 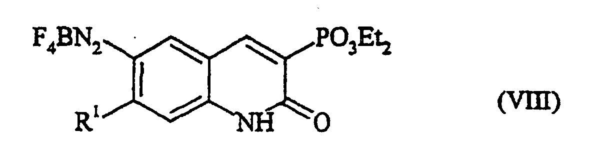 in der R¹ die oben angegebenen Bedeutungen besitzt,
welches man in Gegenwart von Palladium mit einem Boronsäurederivat der Formel (IX) kondensiert: 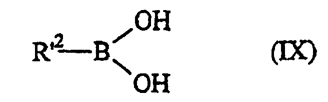 in der R'² eine Aryl- oder Heteroarylgruppe darstellt, wie sie bezüglich der Formel (I) definiert worden ist, welche Heteroarylgruppe über ein Kohlenstoffatom an das Boratom gebunden ist,
zur Bildung der Verbindung der Formel (I/a), einem Sonderfall der Verbindungen der Formel (I): 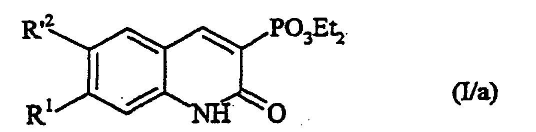 in der R1 und R'² die oben angegebenen Bedeutungen besitzen,
- oder man die Verbindung der Formel (VII) der Einwirkung von 2,5-Dimethoxytetrahydrofuran unterwirft zur Bildung der Verbindung der Formel (I/b), einem Sonderfall der Verbindungen der Formel (I): 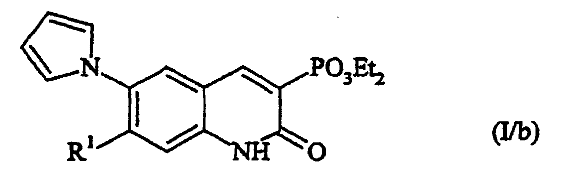 in der R¹ die oben angegebenen Bedeutungen besitzt,
welche Verbindungen der Formeln (I/a) und (I/b) beispielweise in Gegenwart von Trimethylsilanbromid teilweise oder vollständig von den Schutzgruppen befreit werden können zur Bildung der Verbindung der Formel (I/c), einem Sonderfall der Verbindungen der Formel (I): 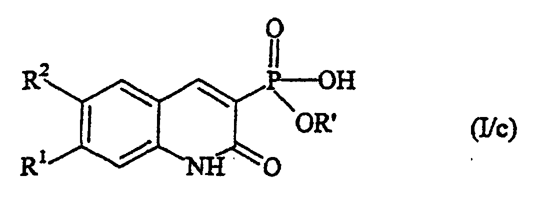 in der R¹ und R² die oben angegebenen Bedeutungen besitzen und R' ein Wasserstoffatom oder eine Ethylgruppe bedeutet,
welche man mit einer Verbindung der Formel (X) kondensieren kann:
R" - Cl (X)
in der R" eine Alkyl-, Aryl- oder Arylalkylgruppe oder eine Gruppe 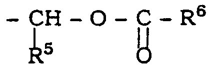 (worin R⁵ und R⁶ die oben angegebenen Bedeutungen besitzen) darstellt,
zur Bildung der Verbindung der Formel (I/d), einem Sonderfall der Verbindungen der Formel (I): 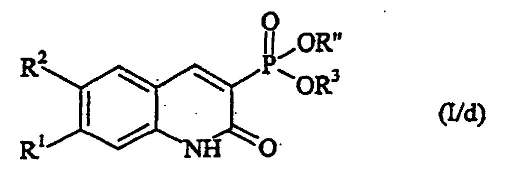 in der R¹, R², R³ und R" die oben angegebenen Bedeutungen besitzen, welche Verbindungen der Formeln (I/a) bis (I/d) die Gesamtheit der Verbindungen der Formel (I) bilden und die man mit Hilfe einer klassischen Trennmethode reinigen kann, die man gewünschtenfalls in ihre Additionssalze mit einer pharmazeutisch annehmbaren Säure oder Base umwandeln kann und die man gegebenenfalls mit Hilfe einer klassischen Trennmethode in ihre Isomeren trennen kann.

8. Pharmazeutische Zubereitungen enthaltend als Wirkstoff mindestens eine Verbindung der Formel (I) nach einem der Ansprüche 1 bis 6 oder eines ihrer Additlonssalze mit einer pharmazeutisch annehmbaren Säure oder Base, allein oder in Kombination mit einem oder mehreren pharmazeutisch annehmbaren Trägermaterialien.

9. Pharmazeutische Zubereitungen nach Anspruch 8 geeignet für die Herstellung eines Arzneimittels zur Behandlung von akuten und chronischen pathologischen Phänomenen, die mit der Hyperaktivierung der Neurotransmissionswege gegenüber anregenden Aminosäuren, wie Zerebralgefäßvorfällen bzw. Schlaganfällen, Gehirn- oder Rückenmarks-Traumata, der Epilepsie, chronischen neurodegenerativen Krankheiten, wie der Alzheimerschen Krankheit. der Schizophrenie, der amyotrophischen Lateralsklerose oder der Huntington-Chorea.

## Claims

1. Compounds of formula (I): 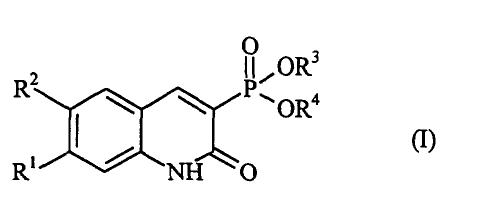 wherein:
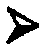 R¹ represents a halogen atom or a trifluoromethyl group,
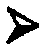 R² represents an aryl or heteroaryl group,
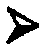 R³ and R⁴, which may be identical or different, represent a hydrogen atom or an alkyl, cycloalkyl, aryl or arylalkyl group or a group 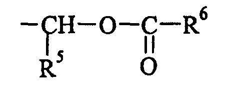 (wherein R⁵ and R⁶, which may be identical or different, represent a hydrogen atom or an alkyl, cycloalkyl or aryl group),
wherein :
- "alkyl" is understood to mean a linear or branched alkyl group containing from 1 to 6 carbon atoms,
- "cycloalkyl" is understood to mean a cyclic alkyl group containing from 3 to 8 carbon atoms,
- "aryl" is understood to mean the groups phenyl, naphthyl or biphenyl, it being possible for those groups to be unsubstituted or substituted by from 1 to 3 groups selected from alkyl, cycloalkyl, alkoxy, polyhaloalkyl, cyano, nitro, amino, alkylamino, dialkylamino, SO₂NR⁷R⁸ (wherein R⁷ and R⁸, which may be identical or different, represent a hydrogen atom or an alkyl, cycloalkyl or aryl group) and halogen atoms,
- "heteroaryl" is understood to mean any mono- or bi-cyclic aromatic group containing from 5 to 10 ring atoms and containing from 1 to 3 hetero atoms selected from oxygen, nitrogen and sulphur, it being possible for the heteroaryl to be attached to the benzene ring that carries it by a carbon atom or by a nitrogen atom when it has one, and for it to be unsubstituted or substituted by from 1 to 3 groups selected from alkyl, cycloalkyl, alkoxy, polyhaloalkyl, cyano, nitro, amino, alkylamino, dialkylamino, SO₂NR⁷R⁸ (wherein R⁷ and R⁸ are as defined hereinbefore) and halogen atoms,
their enantiomers and diastereoisomers and addition salts thereof with a pharmaceutically acceptable acid or base.

2. Compounds of formula (I) according to claim 1 wherein R¹ represents a clorine atom or a group CF₃, their isomers and pharmaceutically acceptable addition salts thereof with an acid or a base.

3. Compounds of formula (I) according to claim 1 wherein R² represents an unsubstituted or substituted naphthyl, phenyl or biphenyl group, their isomers and pharmaceutically acceptable addition salts thereof with an acid or a base.

4. Compounds of formula (I) according to claim 1 wherein R² represents pyrrole or thiophene, their isomers and pharmaceutically acceptable addition salts thereof with an acid or a base.

5. Compounds of formula (I) according to claim 1 wherein R³ and R⁴ simultaneously represent a hydrogen atom, their isomers and pharmaceutically acceptable addition salts thereof with an acid or a base.

6. Compounds of formula (I) according to claim 1 which are {7-chloro-2-oxo-6-[4-(trifluoromethyl)phenyl)-1,2-dihydro-3-quinolyl}phosphonic acid and [7-chloro-6-(4-methylphenyl)-2-oxo-1,2-dihydro-3-quinolyl]phosphonic acid, their isomers and addition salts thereof with a pharmaceutically acceptable base.

7. Process for the preparation of compounds of formula (I) according to claim 1, **characterised in that** there is used as starting material a compound of formula (II) : 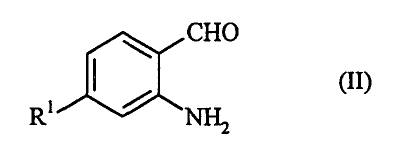 wherein R¹ is as defined for formula (I),
which is condensed, in the presence of a base, such as, for example, pyridine, with a compound of formula (III): 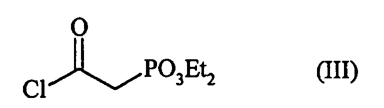 to yield a compound of formula (IV) : 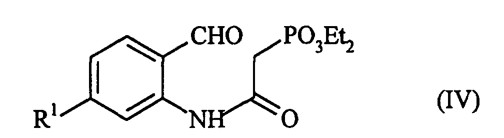 wherein R¹ is as defined hereinbefore,
which is cyclised in the presence of a catalytic amount of piperidine to obtain a compound of formula (V): 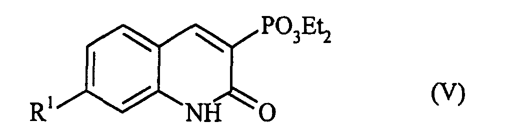 wherein R¹ is as defined hereinbefore,
which is subjected to a mixture of nitric acid and sulphuric acid to yield a compound of formula (VI): 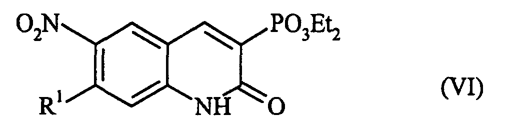 wherein R¹ is as defined hereinbefore,
which is reduced using palladium-on-carbon in the presence of hydrogen or iron in an aqueous alcoholic medium to yield a compound of formula (VII): 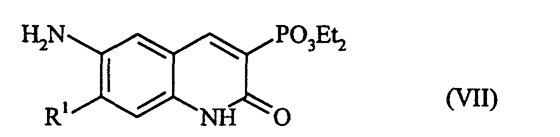 wherein R¹ is as defined hereinbefore,
- which is converted by the action of NaNO₂ and HBF₄ to the corresponding diazonium fluoroborate salt of formula (VIII): 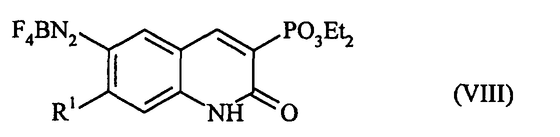
wherein R¹ is as defined hereinbefore,
which is condensed in the presence of palladium with a boronic acid compound of formula (IX) : 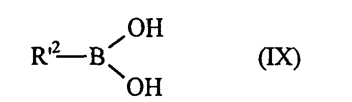 wherein R'² represents an aryl or heteroaryl group as defined for formula (I), the heteroaryl group being attached to the boron atom by a carbon atom,
to yield a compound of formula (I/a), a particular case of the compounds of formula (I): 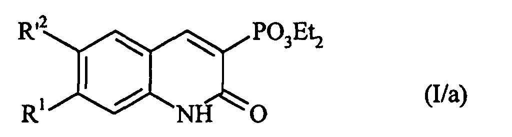 wherein R¹ and R'² are as defined hereinbefore,
- or which compound of formula (VII) is subjected to the action of 2,5-dimethoxytetrahydrofuran to yield a compound of formula (I/b), a particular case of the compounds of formula (I) : 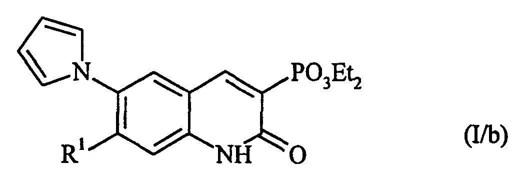
wherein R¹ is as defined hereinbefore,
which compounds of formula (I/a) and (I/b) may be partially or totally deprotected in the presence of, for example, trimethylsilane bromide to yield a compound of formula (I/c), a particular case of the compounds of formula (I) : 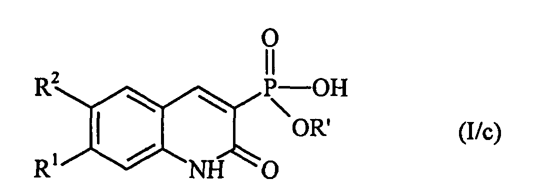 wherein R¹ and R² are as defined hereinbefore and R' represents a hydrogen atom or an ethyl group,
which may be condensed with a compound of formula (x):
R"-Cl (X)
wherein R" represents an alkyl, aryl or arylalkyl group or a group 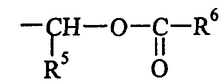 (wherein R⁵ and R⁶ are as defined hereinbefore),
to obtain a compound of formula (I/d), a particular case of the compounds of formula (I): 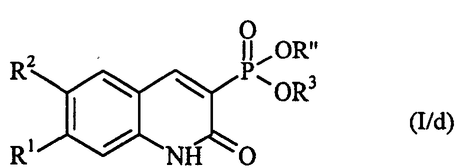 wherein R¹, R², R³ and R" are as defined hereinbefore,
which compounds of formulae (I/a) to (I/d) constitute the totality of the compounds of formula (I), and can be purified according to a conventional separation technique, are converted, if desired, into their addition salts with a pharmaceutically acceptable acid or base, and separated, where appropriate, into their isomers according to a conventional separation technique.

8. Pharmaceutical compositions comprising as active ingredient at least one compound of formula (I) according to any one of claims 1 to 6 or a pharmaceutically acceptable addition salt thereof with an acid or a base, alone or in combination with one or more pharmaceutically acceptable excipients.

9. Pharmaceutical compositions according to claim 8 for use in the manufacture of a medicament for the acute and chronic treatment of pathological phenomena associated with hyperactivation of the neurotransmission paths to the excitatory amino acids, such as cerebrovascular accident, cerebral or spinal traumatism, epilepsy, chronic neurodegenerative diseases such as Alzheimer's disease, schizophrenia, lateral amyotrophic sclerosis or Huntington's chorea.
